# EUROPEAN PATENT APPLICATION

(11) **EP 3 342 424 A1**
(43) Date of publication of application: **04.07.2018**
(21) Application number: 16207293.8
(22) Date of filing: 29.12.2016
(51) Int. Cl.: A61K 47/54

(54) **A SYSTEM OF COLLABORATIVE MODIFIERS AND BINDERS**

(71) Applicant: Yildiz, Marius, 82152 Krailling (DE)
(72) Inventor: Yildiz, Marius, 82152 Krailling (DE)
(74) Representative: Kuttenkeuler, David

(57) **Abstract**

The present invention pertains to a system of collaborative modifiers that are assembled into a multi molecule complex *in vivo* within a cell using bioorthogonal chemical reactions. The system of the invention is composed of multiple binding molecules having a binding affinity to a biological target macromolecule. Each of the binding molecules individually can easily be delivered into a target cell and then, when they bind to their target in the cell, will assemble into a higher multi-molecule complex on the target to increase overall binding specificity, selectivity and strength. Using the invention allows to mimic complex protein-protein interactions, for example the binding of biological macromolecules such as antibodies or receptor ligands with a set of small binding molecules that can be irreversibly assembled during use. The invention is of particular use for therapeutic applications involving intracellular drug targets and/or for crossing the blood brain barrier for targets within the central nervous system and/or for improving therapeutics penetration in tumour tissue. Thus, invention further provides pharmaceutical compositions comprising the components of the system of the invention, and their use in therapy of various diseases. Also provided are methods for the manufacture and design of the collaborative system and system components of the invention.

## Description

### FIELD OF THE INVENTION

The present invention pertains to a system of collaborative modifiers that are assembled into a multi molecule complex *in vivo* within a cell using bioorthogonal chemical reactions. The system of the invention is composed of multiple binding molecules having a binding affinity to a biological target macromolecule. Each of the binding molecules individually can easily be delivered into a target cell and then, when they bind to their target in the cell, will assemble into a higher multi-molecule complex on the target to increase overall binding specificity, selectivity and strength. Using the invention allows to mimic complex protein-protein interactions, for example the binding of biological macromolecules such as antibodies or receptor ligands with a set of small binding molecules that can be irreversibly assembled during use. The invention is of particular use for therapeutic applications involving intracellular drug targets and/or for crossing the blood brain barrier for targets within the central nervous system and/or for improving therapeutics penetration in tumour tissue. Thus, invention further provides pharmaceutical compositions comprising the components of the system of the invention, and their use in therapy of various diseases. Also provided are methods for the manufacture and design of the collaborative system and system components of the invention.

### DESCRIPTION

Over 14,000 protein-protein interactions (PPIs) have been described in humans so far (Roland et al., 2014, Cell 159:1212-1226). The human proteome is estimated to contain over 35,000 domains and 100,000 motifs (Neduva and Russell, 2005, FEBS Lett. 579, 3342-3345). The pharmaceutical industry has successfully developed drugs targeting only a small fraction of the components in the cellular signaling pathways that are misregulated in disease. Recent investigation of drug discovery efforts exposed that of the 15,000-20,000 genes encoded by the human genome, less than 300 have been specifically targeted with small molecules (Over-ington et al., 2006, Nat. Rev. Drug Discov. 2006;5:993-996). About two-thirds of these small molecules are modifying only ten target classes. The size of the classically druggable genome is estimated to be restricted to around 1,500 proteins.

Despite some successes, there have been many failures in discovering effective protein-protein interaction (PPI) inhibitors. Identifying small molecules for PPI inhibition is challenging. As most therapeutically relevant PPIs are intracellular, biologics are less suitable for effective modification of the PPI. The larger size of the biologics limits their delivery to intracellular targets. Developing an efficient PPI inhibitor drug discovery platform represents a significant step toward expanding the druggable proteome.

Antibody-based therapy for cancer is one of the most successful strategies for treating hematological malignancies and solid tumors. Monoclonal antibodies can eliminate cancer cells directly, for example through blocking receptors, by immune-mediated apoptosis of cancer cells or delivery of conjugated cytotoxic drugs to cancer cells. The target epitopes are preferably on the surface of cancer cells. Because of their large size, antibodies are distributed heterogeneously leaving regions of untargeted cells in tumors. This can lead to the increase of drug resistance and subsequent failure of cancer treatment (Thurber et al. Adv Drug Deliv Rev. 2008 September ; 60(12): 1421.). Another common problem is that antibodies can trigger immunogenic responses that can impact safety and pharmacokinetic properties. The immunogenic responses to antibodies reduce the clinical efficacy of antibodies. The immunogenic reaction to antibodies can be grouped to immediate and delayed hypersensitivity responses. The most threatening immediate hypersensitivity response can be severe anaphylactic reactions to the antibody.

Small molecules inhibit an enzyme's activity by occupying most of the space available in the functional cavity of the enzyme. The protein-protein interaction site is in comparison much larger. PPI interface usually encompasses an average area of 1,000-10,000 Å2, which is larger than small-molecule binding sites of 100-600 Å2. A major challenge is that the low ligand efficiencies (LEs) typically associated with small molecule binding at protein surfaces and PPIs raise the barrier to the detection of bona fide low-affinity fragment hits.

Bioorthogonal chemistries are widely used in chemical biology for a myriad of applications such as activity based protein profiling, crosslinking of proteins, monitoring cell proliferation, generation of novel enzyme inhibitors, monitoring the synthesis of newly formed proteins, protein target identification, and studying glycan processing (reviewed in Reyna and Lin 2010, Chem. Commun. (Camb.) 46(10), 1589-1600). Perhaps the most fascinating applications involve using these bioorthogonal chemistries to assemble molecules in the presence of living systems such as live cells or even whole organisms (Baskin et al., 2007, Proc Natl Acad Sci USA, 104, 16793-7; Laughlin et al., 2008, Science, 320, 664-7; Prescher and Bertozzi, 2005, Nat Chem Biol, 1, 13-21; Neef and Schultz, 2009, Angew Chem Int Ed Engl, 48, 1498-500; Ning et al., 2008, Angewandte Chemie-International Edition, 47, 2253-2255). These latter applications require that the chemistry be non-toxic and possess kinetics that allow fast reaction to occur with micromolar concentrations of reagents in a time span of minutes to hours.

WO 2007/110811 discloses an approach to inactivate small molecule drugs using the Staudinger Ligation chemistry. The document proposes to split active small molecular compounds into two parts and to re-connect these compounds in vivo within a cell using the Staudinger chemistry. For this approach, each separated part of the active compound is connected to a Staudinger functional group. Complex binding epitopes, which are present in PPI are not mentioned.

The above problem is solved in a first aspect by a system of collaborative modifiers, comprising at least two or more species of target binding molecule(s), and optionally one or more species of bridging molecule(s).

In some embodiments the problem is solved by a system of collaborative modifiers, comprising at least a first and a second target-binding molecule (species), wherein each target-binding molecule comprises a target binding segment, a connecting segment and optionally a linker, the linker connecting the target-binding segment and the connecting segment, wherein the target-binding segment of the first target-binding molecule mediates binding of the first target-binding molecule to a first binding site, and the target-binding segment of the second target-binding molecule mediates binding of the second target-binding molecule to a second binding site; wherein a connecting segment, when brought into direct contact with another connecting segment, forms a covalent or non-covalent bond between the two connecting segments, and wherein the first and second target binding molecules are designed such that upon binding of the first target-binding molecule to the first binding site, and binding of the second target-binding molecule to the second binding site, the first target-binding molecule and the second target-binding molecule become spatially arranged such, that at least one covalent and/or non-covalent bond is formed connecting the connecting segment of the first target-binding molecule directly or indirectly with the connecting segment of the second target binding molecule, to thereby connect the first and second target binding molecules; and thereby to form an assembled collaborative modifier.

The target binding molecules are in context of the present invention referred to as first, second, third etc. target binding molecules. The prefix "first", "second", "third", shall be understood to individualize each species of target binding molecules in one given possible system of collaborative modifiers in accordance with the invention. The molecular structure of a "first target binding molecule" may however vary between two distinct given systems of collaborative modifiers according to the present invention.

The system of the invention solves the problem because the invention enables the skilled artisan to design a system of small collaborative modifiers which when assembled in vivo are specific for a multi-site protein binding area and function similar to large protein binders such as antibodies or receptor ligands. Since all components of the system of the invention are relatively small and can be taken-up by a cell and then assemble only when being in contact with their target, the invention offers a solution to design modifiers and binders specific for intracellular targets, and/or for crossing the blood brain barrier for targets within the central nervous system and/or for improving therapeutics penetration in tumour tissue. In addition, the invention allows for controlling the point in time of assembly of the system within a cell by for example providing key target binding molecules or bridging molecules separately from the other components of the system.

The "system of collaborative modifiers" in accordance with the invention shall pertain to a combination of several - at least two - species of individual (small) molecular components. Preferably, these components are described herein as target binding molecules, each system comprises at least two target binding molecules. Optionally, in some embodiments the system may further include additional target binding molecules and/or bridging molecules. These components will be described in more detail herein below. When brought into contact with each other and their target, the individual (small) molecular components will spatially assemble in a predetermined pattern due to molecule-target binding specificities and thereby allow forming inter-molecular bonds with each other to form and stabilize a higher molecular complex, referred to herein as "assembled collaborative modifier". The term "collaborative modifier" shall be understood to refer both to assembled collaborative modifier which have a biological impact/activity on their target(s) and to such collaborative modifiers which are simple but specific and selective binders of their target(s) (also called "collaborative binders").

The term "target" in context of the herein disclosed invention shall refer to the molecular structure the assembled collaborative modifier or binder of the invention specifically and selectively interacts with. The target can be a single (preferred) molecular entity or a can be composed of multiple molecular entities. A target of the invention is preferably a macromolecular entity, such as a protein, multimeric protein, nucleic acid, fatty acid, polysaccharide, or a macromolecular fusion thereof. A target of the invention comprises a target binding site area which comprises two or more target binding sites. In some preferred embodiments, the target binding site area is a PPI site, such as a ligand or receptor interaction site. Therefore, a "target binding site" in accordance with the invention is a location on a macromolecular structure which allows for the binding of a single target-binding molecule of the invention as described herein elsewhere. The distance d between at least two target binding sites, with 2d=r, on the target is preferably between 10Å and 5000Å, preferably 10Å to 1000Å, more preferably 10Å to 500Å.

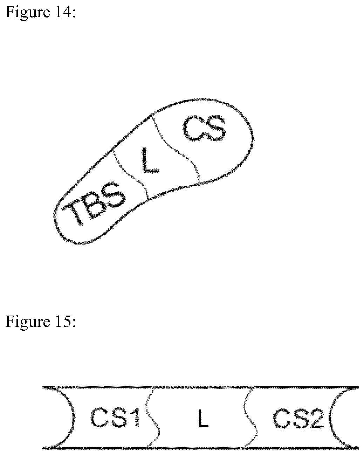

The term "target binding molecule", in context of the present invention, pertains to a molecule, preferably an organic compound, of a pre-determined chemical 3-dimensional structure. The structure of each target-binding molecule of the invention is composed of multiple regions or "segments" which provide particular and distinct functionalities according to the herein described invention. The target-binding molecule of the invention comprises at least one target-binding segment and at least one connecting segment. Optionally the target-binding molecule may further comprise a linker or a linker segment, which connects the target-binding segment and the connecting segment (see figure 14).

A target-binding molecule of the invention is preferably small molecule. Preferably, the target-binding molecule of the invention has a molecular weight of not more than 5000D, preferably of less than 2500D, or less than 1500D.

A "target binding segment" according to the invention shall pertain to a molecular structure motif that provides for an association/dissociation functionality of the target-binding molecule with the target of the invention, preferably providing an interaction with a distinct target binding site of the target. Preferably, the target-binding segment of the invention provides for a specific/selective binding to a target binding site located in the target binding site area of the target of the invention. The binding of the target-binding segment functionality, which refers to the actual atoms involved in the binding to the target, is mediated either by weak interactions such as van-der-Waals forces or hydrogen bonds, or covalent intermolecular bonds. Preferably, each target-binding molecule of the invention has only one single target-binding segment and therefore binds only one single target binding site on the target. In one alternative embodiment of the invention, each target-binding molecule of the invention has one, two, or more target-binding segments and therefore binds to two or more target binding sites on the target.

A "coupling reaction" according to the invention shall refer to the formation of covalent bonds and/or non-covalent bonds between atoms belonging to two different molecules. The term "coupling functional group" refers to specific groups of atoms or bonds within molecules, which are responsible and/or involved in the coupling reaction.

A "connecting segment" according to the invention shall pertain to a molecular structure or motif that provides one of usually two compatible functional groups for a so-called bioorthogonal reaction. Biocompatible or bioorthogonal reactions intended primarily to join substrates of choice with specific biomolecules. In general, bioorthogonal reactions usually join a donor and an acceptor molecule. Bioorthogonal reactions which are suitable for use in living systems generate biological neutral byproducts with no or minimal disturbance to cell function, and are characterized by a high thermodynamic driving force that drives it quickly and irreversibly to high yield of a single reaction product, with high reaction specificity. Therefore, spatial proximity of biorthogonal reaction donor and acceptor will induce the rapid and irreversible bioorthogonal reaction that establishes a bond between the donor and acceptor. Preferred reactions are bioorthogonal reactions. The term "biorthogonal" reaction or chemistry refers to any chemical reaction that can occur in the presence of rich functionalities of living systems/biological media without interfering with native biochemical processes. The two compatible functional groups forming a reaction pair are referred to as a "ligation pair". Many bioorthogonal reactions are known to the skilled artisan. Particularly preferred reactions of this kind are selected from the group consisting of Staudinger Ligation, Nitrone Dipole Cycloaddition, Norbornene Cycloaddition, Oxanor-bornadiene Cycloaddition, Tetrazine Ligation, Cycloaddition, Tetrazole Photoclick Chemistry, and Quadricyclane Ligation. Therefore, a connecting segment of the invention in preferred embodiments will comprise one functional group of a ligation pair of a bioorthogonal reaction described herein before.

A "linker" or "linker segment" in accordance with the present invention may comprise any molecular structure that provides for a mere spatial positioning of the connecting segment at the required 3-dimensional position to allow the coupling reaction to occur. Preferably the linker comprises a simple carbohydrate backbone, such as a straight or branched, substituted or un-substituted C₁-C₂₀ alkyl, heteroalkyl, alkenyl, heteroalkenyl, and/or such as a straight or branched, substituted or un-substituted aryl or heteroaryl. More preferably the linker does not comprise any reactive groups of functionalities that could possibly interfere with the biological reactions in a living cell or organism. The person of skill in the art is able to design a suitable linker for the target binding molecules and the bridging molecules of the inventive system of this disclosure.

In some embodiments of the invention the system further comprising a third target binding molecule, wherein the third target-binding molecule comprises a target binding segment, a first and a second connecting segment, and optionally a first and optionally a second linker, the first linker connecting the target-binding segment and the first connecting segment, and the second linker connecting the target-binding segment and the second connecting segment, and wherein the target-binding segment of the third target-binding molecule mediates binding of the third target-binding molecule to a third binding site, and wherein the first, second and third target binding molecules are designed such, that upon binding of the first target-binding molecule to the first binding site, and binding of the second target-binding molecule to the second binding site, and binding of the third target-binding molecule to the third binding site, the first, second and third target binding molecules become spatially arranged such, that at least one covalent and/or non-covalent bond is formed connecting the connecting segment of the first target-binding molecule directly or indirectly with the first connecting segment of the third target binding molecule, and at least one covalent and/or non-covalent bond is formed connecting the second connecting segment of the third target-binding molecule directly or indirectly with the connecting segment of the second target binding molecule, to thereby form a connection between the first, second and third target binding molecules; and thereby to form an assembled collaborative modifier.

This embodiment provides a collaborative modifier system according to the invention which targets a more complex target binding site area comprising at least three spatially separated target binding sites. From this embodiment it becomes clear that the invention seeks to apply the inventive solution also to even more complex systems comprising at least 4, 5, 6, or more target binding molecules which accordingly will in the assembled collaborative modifier bind to the corresponding 4, 5, 6, or more target binding sites on the target. A target binding molecule of such a "higher order" collaborative modifier system of the invention may further more comprise not only two distinct connecting segments, but also 3, 4, 5 or more connecting segments that each may be linked to the target binding segment via a linker or linker segment. In this way, a system of collaborative modifiers of the invention can when assembled on the target(s) look like a network of "nodes" and "endpoints" which each represent one target binding molecule. In the system each connecting segment comprises as described before one of two compatible coupling functional groups and has in the system at least one, preferably only one, "partner connecting segment" of a different target binding molecule or bridging molecule, wherein the "partner connecting segment" comprises the respective complementary coupling functional group of the ligation pair and will, when brought into spatial proximity according to the present invention form a bond.

In yet another embodiment of the invention, the system may further comprise a bridging molecule, wherein the bridging molecule comprises at least a first and a second connecting segment optionally separated by a linker, and wherein the first and second target binding molecules and the bridging molecule are designed such, that upon binding of the first target-binding molecule to the first binding site, and binding of the second target-binding molecule to the second binding site, the first target-binding molecule and the second target-binding molecule become spatially arranged such that in presence of the bridging molecule a first covalent and/or non-covalent bonds is formed connecting the connecting segment of the first binding molecule directly with the first connecting segment of the bridging molecule, and a second covalent and/or non-covalent bonds is formed connecting the connecting segment of the second binding molecule directly with the second connecting segment of the bridging molecule to thereby form an indirect connection of the first and the second binding molecule; and thereby to form an assembled collaborative modifier.
In yet another embodiment of the invention, the system may further comprise two bridging molecules, wherein the first bridging molecule comprises at least a first and a second connecting segment optionally separated by a first linker, and wherein the second bridging molecule comprises at least a first and a second connecting segment optionally separated by a second linker and wherein the first and second target binding molecules and the first and second bridging molecules are designed such, that upon binding of the first target-binding molecule to the first binding site, and binding of the second target-binding molecule to the second binding site, the first target-binding molecule and the second target-binding molecule become spatially arranged such that in presence of the first and second bridging molecule a first co-valent and/or non-covalent bond is formed connecting the connecting segment of the first binding molecule directly with the first connecting segment of the bridging molecule, and a second covalent and/or non-covalent bonds is formed connecting the second connecting segment of the first bridging molecule directly with the first connecting segment of the second bridging molecule and a third covalent and/or non-covalent bonds is formed connecting the connecting segment of the second binding molecule directly with the second connecting segment of the second bridging molecule to thereby form an indirect connection of the first and the second binding molecule; and thereby to form an assembled collaborative modifier. This embodiment is useful in a situation where a long distance separates two binding sites on a target.

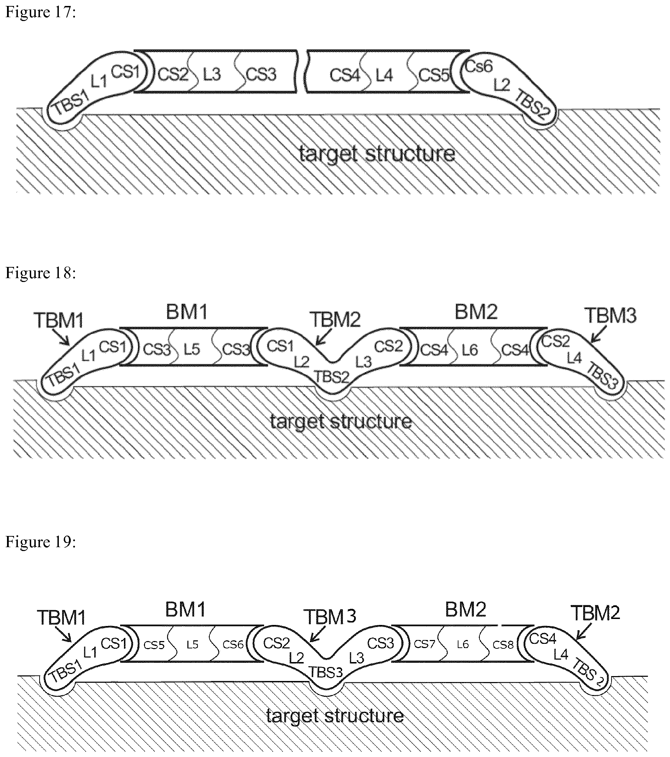

In yet a further embodiment of the invention, the system comprises a first and a second bridging molecule and a third target binding molecule, the third target binding molecule comprising a target binding segment, a first and a second connecting segment, and optionally a first and optionally a second linker, the first linker connecting the target binding segment and the first connecting segment, and the second linker connecting the target binding segment and the second connecting segment, and wherein the target binding segment of the third target-binding molecule mediates binding of the third target binding molecule to a third binding site, wherein the first, second and third target binding molecules and the first and second bridging molecule are designed such, that upon binding of the first target-binding molecule to the first binding site, and binding of the second target binding molecule to the second binding site, and binding of the third target-binding molecule to the third binding site, the first, second and third target binding molecules become spatially arranged such, that in presence of the first and second bridging molecules a first covalent and/or non-covalent bond is formed connecting the connecting segment of the first target binding molecule directly with the first connecting segment of the first bridging molecule, and a second covalent and/or non-covalent bonds is formed connecting the first connecting segment of the third target binding molecule directly with the second connecting segment of the bridging molecule to thereby form an indirect connection of the first and the third target binding molecule; and that at a third covalent and/or non-covalent bond is formed connecting the second connecting segment of the third target binding molecule directly with the first connecting segment of the second bridging molecule, and a fourth covalent and/or non-covalent bond is formed connecting the second connecting segment of the second bridging molecule with the connecting segment of the second target binding molecule to thereby form an indirect connection of the third and the second target binding molecule; to thereby form an indirect connection between the first, second and third target binding molecule; and thereby to form an assembled collaborative modifier. In some embodiments it is preferred that any two of: the connecting segments of the first, the second and the third target binding molecules do not form a bond when brought into contact with each other, preferably, wherein the connecting segment of the first target binding molecule does not bond with a connecting segment of the second bridging molecule, and/or wherein the connecting segment of the second target binding molecule does not bond with a connecting segment of the first bridging molecule, and/or wherein the first connecting segment of the third binding molecule does not bond with a second connecting segment of the third binding molecule, and wherein the first connecting segment of the third binding molecule does not bond with a connecting segment of the second bridging molecule, and wherein the second connecting segment of the third binding molecule does not bond with a connecting segment of the first bridging molecule (see figure 19).

In some embodiments of the invention it may be preferred that in a system of collaborative binders comprising two target binding molecules and at least one bridging molecule between them, or at least one additional third target binding molecule located on the target between the first two target binding molecules, then it is preferably that the connecting segments of the two target molecules comprise incompatible coupling functional groups and therefore do not form a bond when brought into contact with each other. The term "incompatible coupling functional group" in this context refers to two functional groups of two distinct bioorthogonal coupling pairs as described above, and which therefore cannot undergo a coupling reaction.

The bond formed between two connecting segments is preferably a covalent bond, preferably an irreversible covalent bond according to the bond formed between the aforementioned coupling reaction ligation pairs.

In a preferred embodiment of the invention, the individual target binding molecule and/or bridging molecule of the invention has not a biological function or activity of its own, meaning it does not have a biological activity outside of the context of the system of the invention.

In one aspect of the invention each target binding molecule of the collaborative system has a dissociation constant k_{D}[free] describing the binding affinity of an unbound target binding molecule to its target binding site, whereas k_{D}[assembled] describes the binding affinity of the same target binding molecule when being in the assembled collaborative modifier. According to preferred embodiments of the invention the kD[free] is greater than kD[assembled], in other words the target binding molecule has a higher target binding affinity in the assembled state compared to the free unbound state.

It is understood that the invention shall not be bound to any specific numbers of target binding molecules or bridging molecules. Depending on the complexity of the target and the number of target binding sites located thereon, the person of skill may design based on the disclosure of the invention more complex collaborative modifiers, for example comprising 4, 5, 6 or more target binding molecules, which may include one or more target binding molecules with two or more connecting segments, and wherein the collaborative modifier may include any number of bridging molecules. Using the herein described system of modifiers, the person of skill can design new collaborative modifiers for any target binding site layout.

### Pharmaceutical Compositions

Another aspect of the invention then pertains to a pharmaceutical composition comprising any one or a combination of the herein disclosed components of the system of the invention, and a pharmaceutical acceptable carrier and/or excipient. The pharmaceutical composition is preferably for use in the prevention or treatment of a disease.

A preferred disease treatable with the collaborative modifier of the invention is a disease where a target molecule of the collaborative modifier of the invention (the drug target) is located intracellular.

In some embodiments of the invention the pharmaceutical composition comprises at least one, preferably two, more preferably three or more, or all target binding molecule(s) of the herein elsewhere described system of collaborative modifiers.

In other embodiments of the invention, the pharmaceutical composition comprising one or more target binding molecules does not comprise a bridging molecule.

In other embodiments of the invention the pharmaceutical composition comprises one, two, or more bridging molecule(s) of the herein before described system of collaborative modifiers of the invention. In this case it may be preferred that the pharmaceutical composition at the same time does not comprise a target binding molecule of the invention.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include oral, parenteral, e.g., intrathecal, intra-arterial, intravenous, intradermal, subcutaneous, transdermal (topical) and transmucosal administration.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminium monostearate and gelatin.

Sterile injectable solutions can be prepared by incorporating the active compound (e.g., a neuregulin) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavouring agent such as peppermint, methyl salicylate, or orange flavouring.

Furthermore, the compounds of the invention can be administrated rectally. A rectal composition can be any rectally acceptable dosage form including, but not limited to, cream, gel, emulsion, enema, suspension, suppository, and tablet. One preferred dosage form is a suppository having a shape and size designed for introduction into the rectal orifice of the human body. A suppository usually softens, melts, or dissolves at body temperature. Suppository excipients include, but are not limited to, theobroma oil (cocoa butter), glycerinated gelatin, hydrogenated vegetable ails, mixtures of polyethylene glycols of various molecular weights, and fatty acid esters of polyethylene glycol.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

It is especially advantageous to formulate oral, rectal or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

As mentioned before, the herein described components of the system of collaborative modifiers and the pharmaceutical compositions are preferably for use in medicine, for example in the treatment of a disease in a subject. The invention is based on the fact that each species of target binding molecules of the invention is as such insufficient to modulate a biological function, preferably within a cell. However, the combined binding of all target binding molecules of the system is permanently stabilized via the coupling reaction of the connecting segments of the target binding molecules and, optionally in some embodiments, the bridging molecules. Therefore, the individual components of the system have a very low biologic activity and can be easily administered without toxic side effects. Furthermore, since the therapeutic effect is only provided by the assembled collaborative modifier, the system of the invention provides a therapeutic with less off target effects. Furthermore, since the assembly of the modifier is dependent on the presence of all essential components for forming the connection between all target binding molecules of the system, the separate administration of one key component can be used to avoid unintentional complex formation.

Therefore, according to some preferred embodiments a treatment of a subject with a system of the invention containing at least three target binding molecules comprises a separate administration of components (i) and (ii), wherein component (i) is the first and/or second target binding molecule, and component (ii) is the third (interjacent) target binding molecule.

In other preferred embodiments of the invention, the treatment comprises a separate administration of the target binding molecule(s) and the bridging molecule(s).

Also provided is a method for manufacturing or designing the system of collaborative modifiers or binders as described herein elsewhere.

The present invention will now be further described in the following examples with reference to the accompanying figures and sequences, nevertheless, without being limited thereto. For the purposes of the present invention, all references as cited herein are incorporated by reference in their entireties. **In the Figures:**
**Figure 1****:** Target binding molecule (TBM) 1. ZINC01535869 is the target binding structure (TBS1) bound with the linker (L2) to the connecting segment (CS1) and bound to the linker (L3) to the connecting segment (CS2).
**Figure 2****:** TBM2 consists of the TBS2 directly bound with the CS1. A linker was not necessary.
**Figure 3****:** Target binding molecule (TBM) 3. ZINC02169852 is the target binding structure (TBS3) bound with the linker (L4) to the connecting segment (CS2).
**Figure 4****:** TBM1, TBM2, and TBM3 bound to distinct binding sites on the surface of Keap1. TBM1 target binding molecule 1; TBM2 target binding molecule 2; TBM3 target binding molecule 3; CS1 connecting segment 1; CS2 connecting segment 2.
**Figure 5****:** Consists of the linker (L5) flanked at its ends by 2 connecting segments (CS3). The connecting segments are separated by the linker (L5). The Linker L5 is selected from a fragment base in order to align TBM1 and TBM2 after binding of the BM1 to the target binding molecules TBM1 and TBM2.
**Figure 6****:** Consists of the linker (L6) flanked at its ends by 2 connecting segments (CS4).
**Figure 7****:** The collaborative modifier (CM). The bridge molecule 1 (BM1) forms covalent bonds with the target binding molecules TBM1 and TBM2, and the BM2 forms covalent bonds with the TBM2 and TBM3. Thus BM1, BM2 and TBM1, TBM2 and TBM3 from together after binding of the bridge molecules the collaborative modifier (CM).

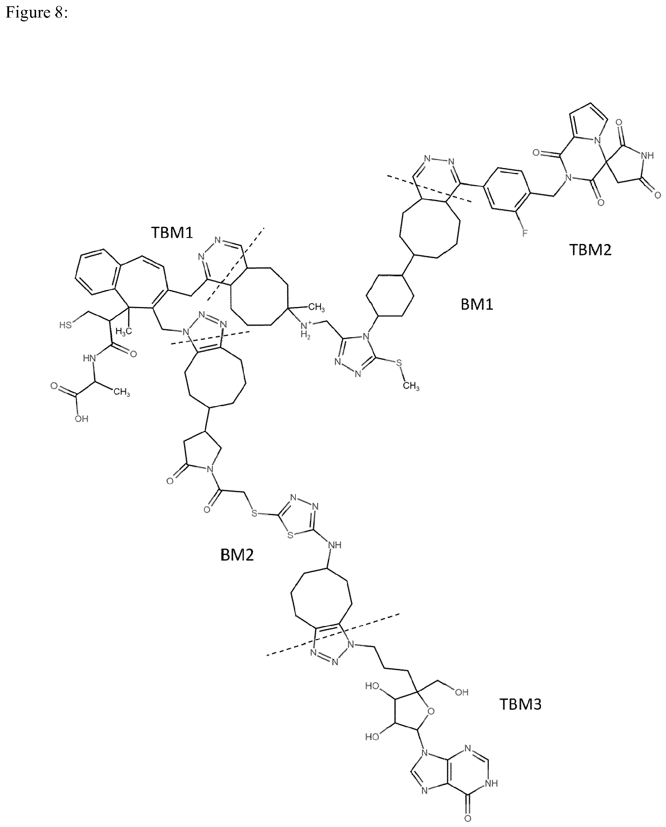

**Figure 8****:** The collaborative modifier (CM).
**Figure 9****:** Target binding molecule 1 (TBM1). This molecule is a combination of the target binding segment TSB1 (PDB entry: 0QY) and the connecting structure tetrazine without a linker between them.
**Figure 10****:** Target binding molecule 2 (TBM2). This molecule is a combination of the target binding segment TSB2 (PDB entry: 21J) and the connecting structure tetrazine without a linker between them.
**Figure 11****:** Bridge molecule (BM). The BM consists of the linker (L3) and two connecting segments (CS2).
**Figure 12****:** The collaborative modifier (CM) is formed after the bridge molecule (BM) binds covalently to the target binding molecule 1 (TBM) and the target binding molecule 2 (TBM2).
**Figure 13****:** 3D depiction of the collaborative modifier (CM). The bridge molecule is covalently attached to the TBM1 and TBM2.
**Figure 14****:** shows a target binding molecule (TBM) of the invention. TBS= target binding site, L=linker, CS=connecting segement.
**Figure 15****:** shows a bridging molecule (BM) of the invention.
**Figure 16****:** shows an embodiment of a collaborative modifier where two TBM are connected with a BM. A: general architecture of the embodiment of the collaborative modifier; B: the embodiment where the BM and the TBM1 and TBM2 are coupled via the same kind of coupling groups; C: the embodiment where the BM and the TBM1 and the BM and the TBM2 are coupled via different coupling groups.
**Figure 17****:** shows an embodiment where two BM are used to connect two TBMs.
**Figure 18****:** shows an embodiment with three TBM which are interconnected with two BM.
**Figure 19****:** shows an embodiment with three TBM which are interconnected with two BM, where each coupling pairs are provided by different functional groups.

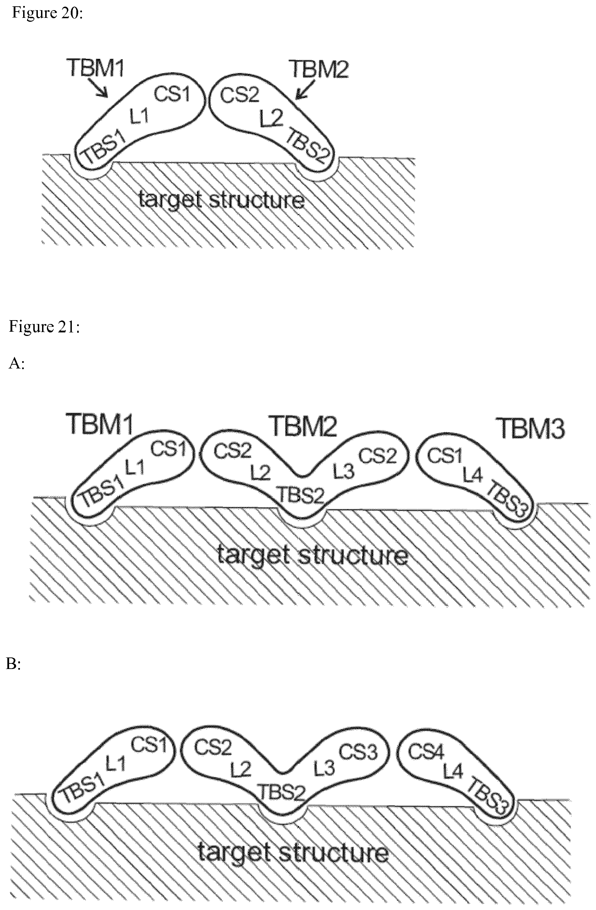

**Figure 20****:** shows a simple embodiment of the invention where two TBM are used for a collaborative modifier.
**Figure 21****:** A shows an embodiment with three TBM using for each connecting segment the same kind of coupling pairs; B shows an embodiment with three TBM using for each connecting segment a different kind of coupling pairs.

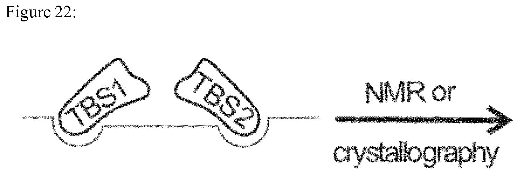

**Figure 22****:** Shows the procedure for the design of a collaborative modifier of the invention. Two structures that are known as binders of different target binding sites on the target are structurally analyzed via NMR or crystallography to identify their positioning on the target. Based on the structural information suitable linker and connecting segments for both TBS can be designed to arrive at a collaborative modifier of example Figure 20.

### EXAMPLES

### EXAMPLE 1

The Keap1-Nrf2-ARE pathway counteracts oxidative stress. Increasing the activity of Nrf2 could be of therapeutic use in many human diseases, for example cancer, neurological diseases, airway disorders, cardiovascular diseases, diabetes, inflammatory bowel disease, and autoimmune diseases (Dhulfiqar AA, et al. Discovery of direct inhibitors of Keap1-Nrf2 protein-protein interaction as potential therapeutic and preventive agents. Acta Pharmaceutica Sinica B 2015; 5(4):285-299). Keap1 (Kelch ECH associating protein 1) is a repressor protein that binds to Nrf2 and promotes its degradation by the ubiquitin proteasome pathway.

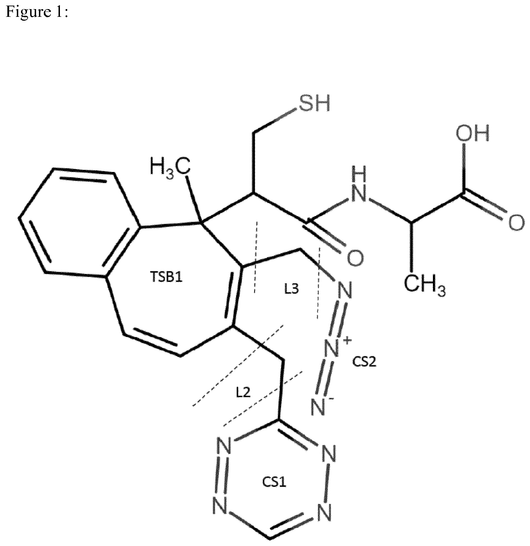

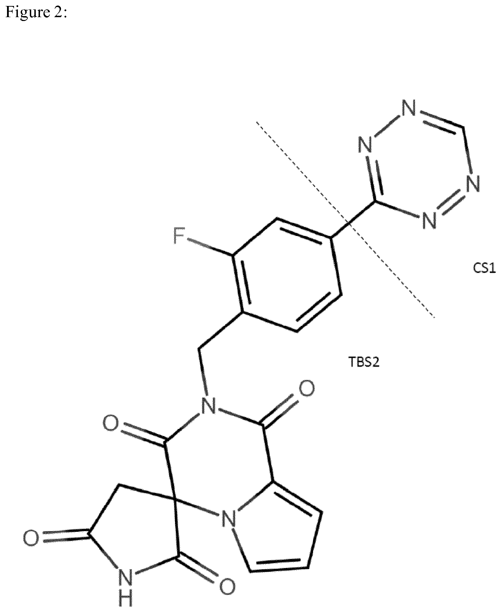

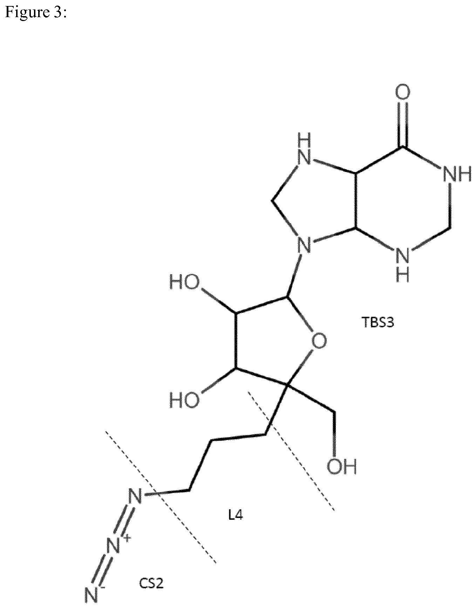

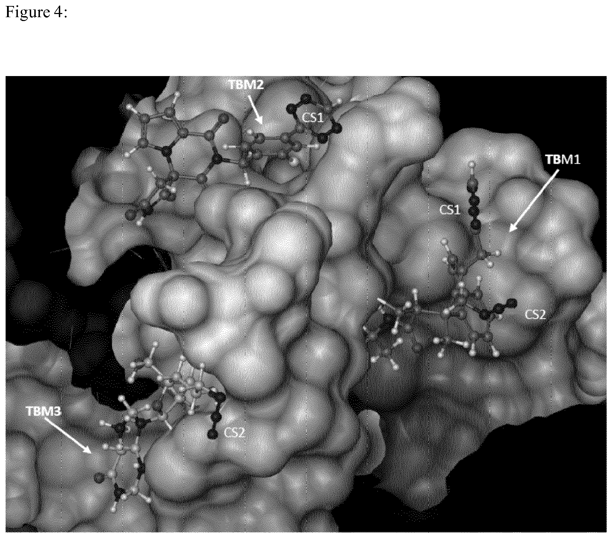

This example uses the general draft of the collaborative modifier (CM) from figure 18. Given a reference structure of the kelch domain of Keap1 (protein data bank entry: 1U6D), a structure-based virtual screening was performed to select small molecules likely to interact with the surface of the residues 368 to 400. For the in-silico screening a database of 10 million compounds derived from ZINC15 (Sterling and Irwin, J. Chem. Inf. Model, 2015 http://pubs.acs.org/doi/abs/10.1021/acs.jcim.5b00559) was used. Docking was performed with FlexX (part of the software package LeadIT version 2.1.9 from BioSolveIT GmbH, St. Augustin, Germany). The chemical structures of the target binding segments (TBS) were modified with a molecular editor (Avogadro Version 1.2.0; Hanwell, et al. "Avogadro: An advanced semantic chemical editor, visualization, and analysis platform" Journal of Cheminformatics 2012, 4:17). A linker and a connecting segment was attached to the original structure. The linker was placed at the TBS so that the connecting segment (CS) of the target binding molecule (TBM) could be connected via a bridge molecule to its corresponding TBM to form the CM. The geometry of the modified compounds was optimized in the next step with MMFF94 (Merck molecular force field. I. Basis, form, scope, parameterization, and performance of MMFF94, Thomas A. Halgren, J. Comp. Chem.; 1996; 490-519). These target binding molecules were then retested and docked again with the surface of Keap1. Three modified compounds which were predicted to interact favorably with the Keap1 surface were selected: (1) ZINC01535869 was used as TSB1 of TBM1 (fig.1); (2) ZINC00598588 was used as TSB2 (fig. 2); (3) ZINC02169852 was used as TSB3 (fig. 3). These three compounds were binding to distinct target binding sites on the surface of Keap1 (fig. 4).

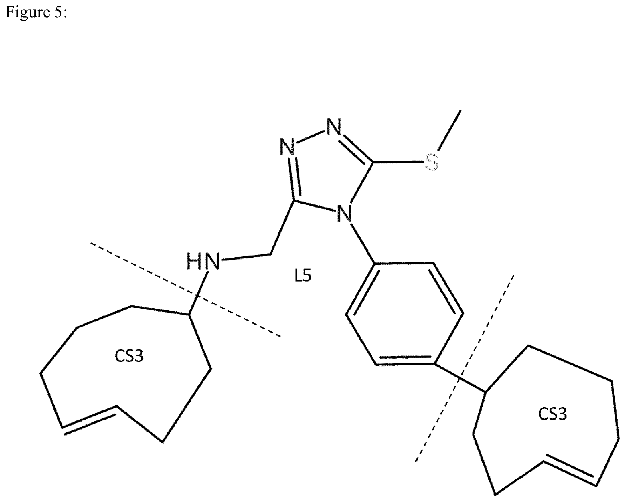

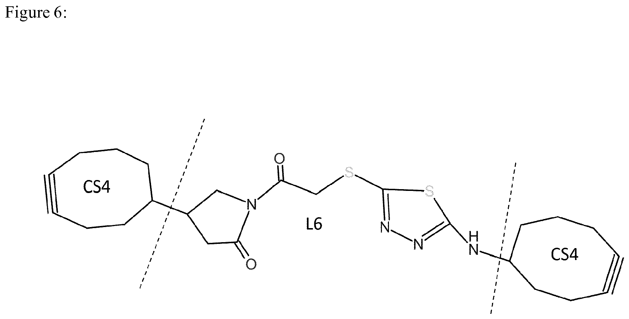

The linker parts of the bridge molecules 1 and 2 (BM1 and BM2, fig. 5 and fig. 6) were selected automatically by the ReCore software (part of the software package LeadIT version 2.1.9 from BioSolveIT GmbH, St. Augustin, Germany). The programme searches a fragment library for fragments which can connect molecules retaining the 3-dimensional spatial orientation of the vectors of the molecules (target binding molecules) which should be connected with bridge molecules to each other. The best fit fragment was selected. Best fit was defined as the fragment (1) which causes the least deviation to the spatial orientation vectors of the target binding molecules and (2) at the same time has affinity (non-covalent binding) for the drug target (Keap1 surface). The affinity of the linker of the BM helps to align the BM with the other participating molecules of the collaborative modifier (CM).

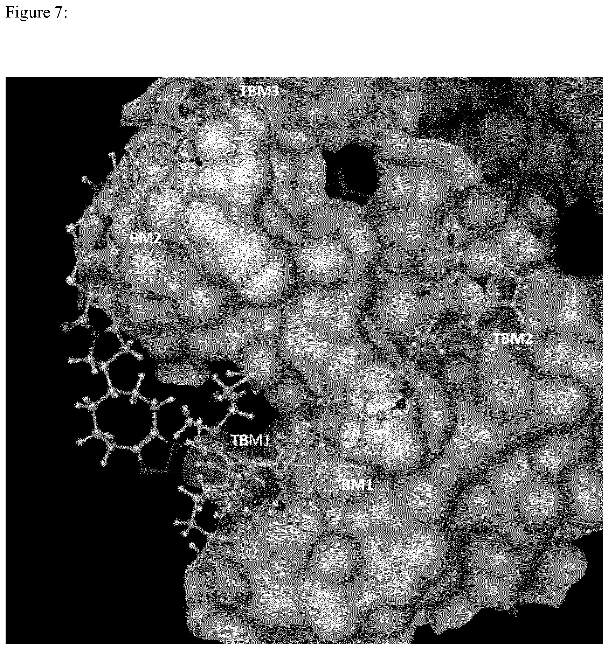

The trans-cyclooctenes (TCO) of the both CS3 of the bridge molecule 1 (BM1) react spontaneously and selectively with the 1,2,4,5-tetrazines of the CS1 of the target binding molecules TBM1 and TBM2, forming covalent bonds between BM1 with TBM1 and between BM1 with TBM2. The alkynes of the CS4 of the BM2 react spontaneously with the triazoles of the CS2 of TBM1 and the CS2 of TBM3, forming covalent bonds between BM2 with TBM1 and between BM2 with TBM3. Thus BM1, BM2 and TBM1, TBM2 and TBM3 from together after binding of the bridge molecules the CM (fig. 7).

The components of the CM: TBM1, TBM2, TBM3, BM1, and BM2 can be structurally changed by a person skilled in the art to improve the affinity of the CM towards the drug target surface of Keap1. The affinity of the CM to the target surface of Keap1 can, for example, be measured with Biacore 4000 (GE Healthcare, Chicago, Illinois, United States). The Biacore 4000 can be used by the person skilled in the art to measure also the inhibition of the protein-protein interaction between Keap1 and Nrf2 by the CM. The affinity of the CM towards the target surface of Keap1 and PPI inhibition data can be used to further optimize the chemical structure of the CM components.

### EXAMPLE 2

Inhibition of K-Ras activity in tumors or cancer cell lines has been shown to result in the reversal of the malignant phenotype and suppression of tumorigenicity in human cancer cells (Podsypanina et al.. Proc Natl Acad Sci. 2008; 105:5242-5247).

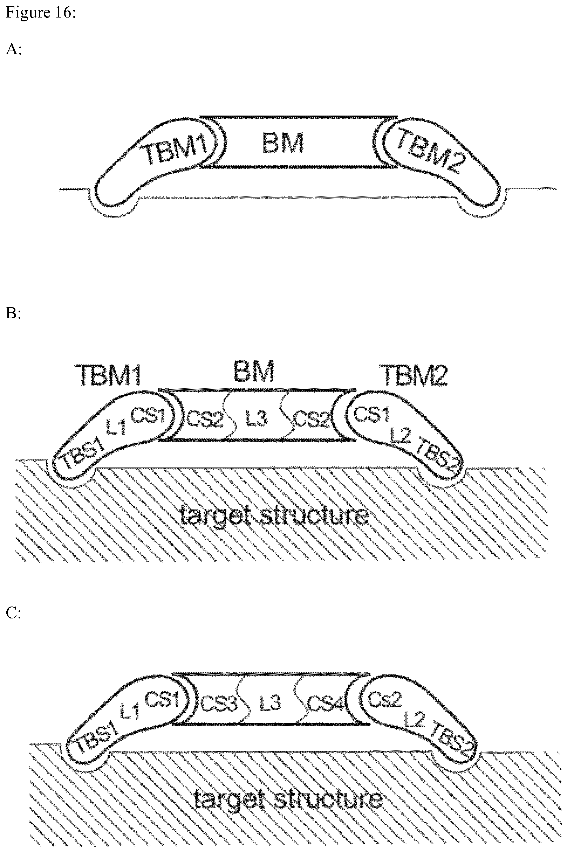

This example uses the general draft of the collaborative modifier (CM) from figure 16B. In the first step two known molecules which bind to KRas were selected as target binding structures (TBS). The binding sites are on the surface of KRas which interacts with SOS. Thus, these molecules are orthosteric inhibitors of the Ras-SOS protein-protein-interaction (PPI).

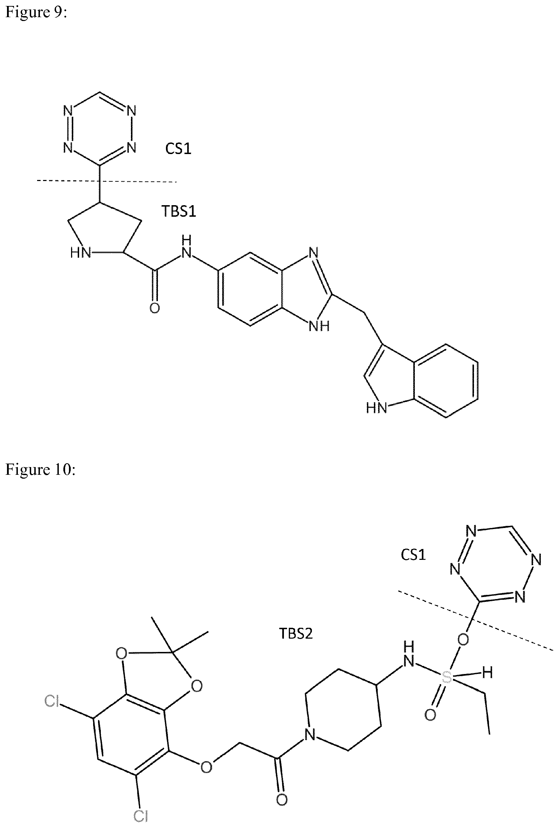

The protein data bank (PDB) entry of target binding segment 1 (TBS1) is "0QY". The crystal structure of TBS1 bound to KRas has the PDB entry number "4EPY". The ligand data was retrieved from PDB and modified with molecule editing software. Tetrazine was attached as CS1 to the TBS1 so that TBM1 (see fig. 9) can be connected with a bridge molecule (BM) to the second target binding molecule (TBM2).

The protein data bank (PDB) entry of TBS2 is "21J". The crystal structure of TBS2 bound to KRas has the PDB entry number "4M10" The ligand data was retrieved from PDB and modified with molecule editing software. Tetrazine was attached as CS1 to the TBS2 so that TBM2 (see fig. 10) can be connected with a bridge molecule (BM) to the first target binding molecule (TBM1).

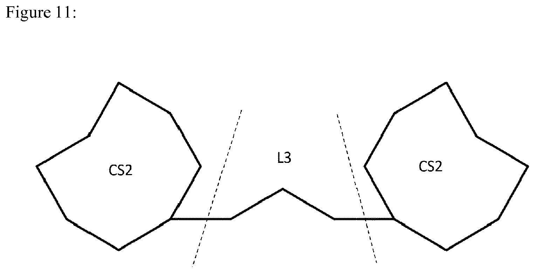

The linker of the BM L3 (fig. 11) was selected in order to connect TBM1 and TBM2 with the least deviation to the spatial orientation vectors of the target binding molecules (TBM1 and TBM2) when they are bound to their corresponding binding sites on the surface of KRas without being connected to each other by the BM. The geometry of the modified compounds TBM1, TBM2 and BM was optimized with MMFF94 (Merck molecular force field. I. Basis, form, scope, parameterization, and performance of MMFF94, Thomas A. Halgren, J. Comp. Chem.; 1996; 490-519).

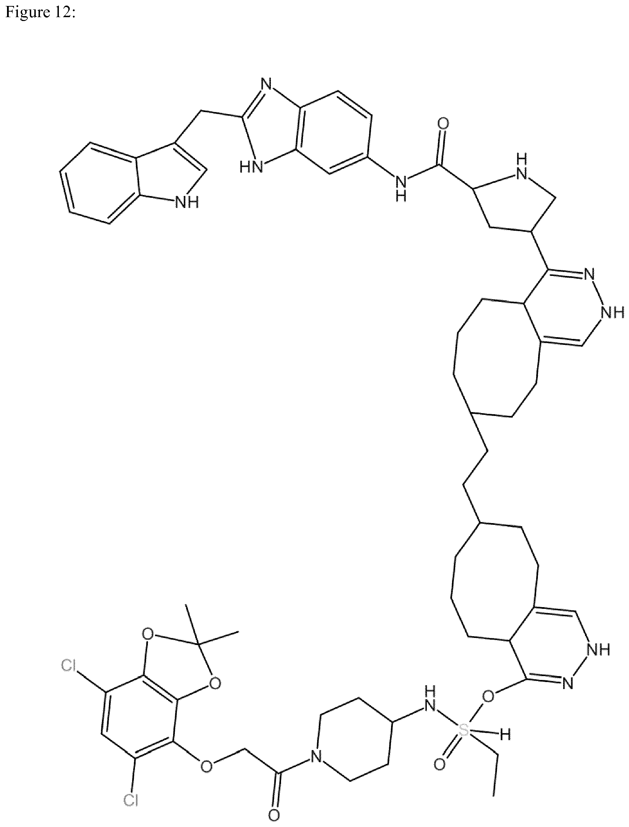

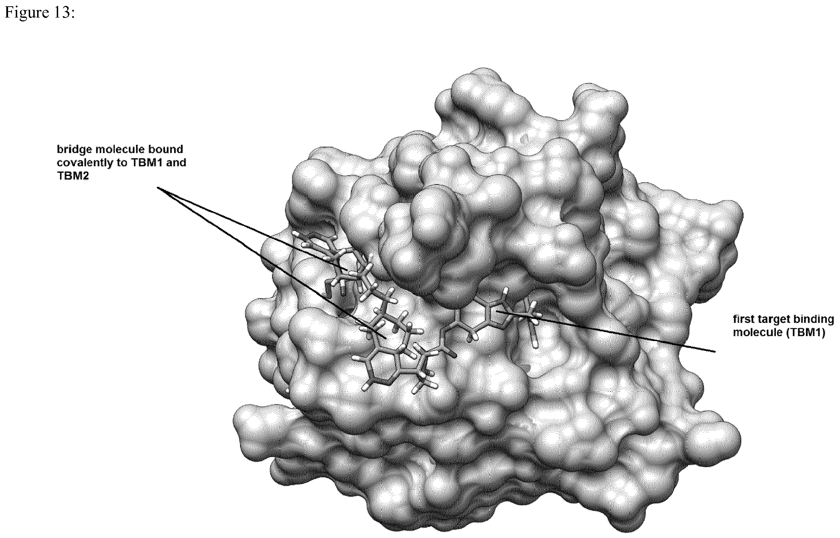

The trans-cyclooctenes (TCO) of the both CS2 of the BM react spontaneously and selectively with the 1,2,4,5-tetrazines of the CS1 of the target binding molecules TBM1 and TBM2, forming covalent bonds between BM with TBM1 and between BM with TBM2 (fig. 12 and fig.13).

The components of the CM: TBM1, TBM2 and BM can be structurally changed by a person skilled in the art to improve the affinity of the CM towards the drug target surface of KRas. The affinity of the CM to the target surface of KRas can, for example, be measured with Biacore 4000 (GE Healthcare, Chicago, Illinois, United States). The Biacore 4000 can be used by the person skilled in the art to measure also the inhibition of the protein-protein interaction (PPI) between KRas and SOS by the CM. The affinity and PPI inhibition data can be used to further optimize the chemical structure of the CM components

## Claims

1. A system of collaborative modifiers, comprising at least a first and a second target binding molecule, wherein each target-binding molecule comprises a target binding segment, a connecting segment and optionally a linker, the linker connecting the target-binding segment and the connecting segment,
wherein the target-binding segment of the first target-binding molecule mediates binding of the first target-binding molecule to a first binding site, and the target-binding segment of the second target-binding molecule mediates binding of the second target-binding molecule to a second binding site;
wherein a connecting segment, when brought into direct contact with another connecting segment, forms a covalent or non-covalent bond between the two connecting segments, and
wherein the first and second target binding molecules are designed such that upon binding of the first target-binding molecule to the first binding site, and binding of the second target-binding molecule to the second binding site, the first target-binding molecule and the second target-binding molecule become spatially arranged such, that at least one covalent and/or non-covalent bond is formed connecting the connecting segment of the first target-binding molecule directly or indirectly with the connecting segment of the second target binding molecule, to thereby connect the first and second target binding molecules; and thereby to form an assembled collaborative modifier.

2. The system according to claim 1, further comprising a third target binding molecule,
wherein the third target-binding molecule comprises a target binding segment, a first and a second connecting segment, and optionally a first and optionally a second linker, the first linker connecting the target-binding segment and the first connecting segment, and the second linker connecting the target-binding segment and the second connecting segment, and
wherein the target-binding segment of the third target-binding molecule mediates binding of the third target-binding molecule to a third binding site, and
wherein the first, second and third target binding molecules are designed such, that upon binding of the first target-binding molecule to the first binding site, and binding of the second target-binding molecule to the second binding site, and binding of the third target-binding molecule to the third binding site, the first, second and third target binding molecules become spatially arranged such, that at least one covalent and/or non-covalent bond is formed connecting the connecting segment of the first target-binding molecule directly or indirectly with the first connecting segment of the third target binding molecule, and at least one covalent and/or non-covalent bond is formed connecting the second connecting segment of the third target-binding molecule directly or indirectly with the connecting segment of the second target binding molecule, to thereby form a connection between the first, second and third target binding molecules; and thereby to form an assembled collaborative modifier.

3. The system according to claim 1, further comprising a bridging molecule, wherein the bridging molecule comprises at least a first and a second connecting segment optionally separated by a linker, and
Wherein the first and second target binding molecules and the bridging molecule are designed such, that upon binding of the first target-binding molecule to the first binding site, and binding of the second target-binding molecule to the second binding site, the first target-binding molecule and the second target-binding molecule become spatially arranged such that in presence of the bridging molecule a first covalent and/or non-covalent bonds is formed connecting the connecting segment of the first binding molecule directly with the first connecting segment of the bridging molecule, and a second covalent and/or non-covalent bonds is formed connecting the connecting segment of the second target binding molecule directly with the second connecting segment of the bridging molecule to thereby form an indirect connection of the first and the second binding molecule; and thereby to form an assembled collaborative modifier.

4. The system according to claim 3, comprising a first and a second bridging molecule and a third target binding molecule, the third target-binding molecule comprising a target binding segment, a first and a second connecting segment, and optionally a first and optionally a second linker, the first linker connecting the target-binding segment and the first connecting segment, and the second linker connecting the target-binding segment and the second connecting segment, and wherein the target-binding segment of the third target-binding molecule mediates binding of the third target-binding molecule to a third binding site,
Wherein the first, second and third target binding molecules and the first and second bridging molecule are designed such, that upon binding of the first target-binding molecule to the first binding site, and binding of the second target-binding molecule to the second binding site, and binding of the third target-binding molecule to the third binding site, the first, second and third target binding molecules become spatially arranged such, that in presence of the first and second bridging molecules a first covalent and/or non-covalent bond is formed connecting the connecting segment of the first binding molecule directly with the first connecting segment of the first bridging molecule, and a second covalent and/or non-covalent bonds is formed connecting the first connecting segment of the third binding molecule directly with the second connecting segment of the bridging molecule to thereby form an indirect connection of the first and the third target binding molecule; and that at a third covalent and/or non-covalent bond is formed connecting the second connecting segment of the third binding molecule directly with the first connecting segment of the second bridging molecule, and a fourth covalent and/or non-covalent bond is formed connecting the second connecting segment of the second bridging molecule with the connecting segment of the second binding molecule to thereby form an indirect connection of the third and the second target binding molecule; to thereby form an indirect connection between the first, second and third target binding molecule; and thereby to form an assembled collaborative modifier.

5. The system according to claim 4, wherein any two of: the connecting segments of the first, the second and the third target binding molecule do not form a bond when brought into contact with each other, preferably, wherein the connecting segment of the first binding molecule does not bond with a connecting segment of the second bridging molecule, and/or wherein the connecting segment of the second target binding molecule does not form a bond with a connecting segment of the first bridging molecule.

6. The system according to any of the preceding claims, wherein the bond formed between two connecting segments is a covalent bond, preferably an irreversible covalent bond.

7. The system according to any one of the preceding claims, wherein two connecting segments that are capable of forming a bond between each other are two non-identical components of a connecting segment system, comprising one donor segment and one acceptor segment, wherein when a donor segment and an acceptor segment are brought into contact with each other a bond is formed between them, and wherein when two donor or two acceptor segments are brought into contact with each other no bond is formed between them.

8. The system according to any one of the preceding claims, wherein the individual target binding molecule and/or bridging molecule has not a biological function or activity of its own.

9. The system according to any one of the preceding claims, wherein any two connecting segments that form a bond with each other, comprise compatible functional groups of a bioorthogonal chemical reaction, preferably selected from the group consisting of such as the Staudinger Ligation, Nitrone Dipole Cycloaddition, Norbornene Cycloaddition, Oxanorbornadiene Cycloaddition, Tetrazine Ligation, Cycloaddition, Tetrazole Photoclick Chemistry, and Quadricyclane Ligation.

10. The system according to any one of the preceding claims, wherein the dissociation constant of each binding molecule to the target is lower when the binding molecule is in the assembled collaborative modifier compared to the non-assembled (free) molecule.

11. A pharmaceutical composition comprising a pharmaceutical carrier and/or excipient in admixture with at least one binding molecule and/or bridging molecule of a system according to any one of the preceding claims.

12. The pharmaceutical composition according to claim 11, comprising the first and/or the second and/or the third target binding molecule of the system according to any one of claims 1 to 10.

13. The pharmaceutical composition according to claim 11, comprising the first and/or second bridging molecule of the system according to any one of claims 3 to 10.

14. The target binding molecule and/or bridging molecule of the system according to any one of claims 1 to 10, for use in the treatment of a disease in a subject.

15. A method of producing a system of collaborative modifiers according to any one of claims 1 to 10.
